(19) Europäisches Patentamt
European Patent Office
Office européen des brevets

(11) Veröffentlichungsnummer: **0 204 231**
**A2**

(12)

# EUROPÄISCHE PATENTANMELDUNG

(21) Anmeldenummer: **86107042.3**

(51) Int. Cl.⁴: **C 07 D 213/64**

(22) Anmeldetag: **23.05.86**

(30) Priorität: **05.06.85 DE 3520097**

(43) Veröffentlichungstag der Anmeldung:
**10.12.86 Patentblatt 86/50**

(84) Benannte Vertragsstaaten:
**CH DE FR GB IT LI**

(71) Anmelder: **BAYER AG**
**Konzernverwaltung RP Patentabteilung**
**D-5090 Leverkusen 1 Bayerwerk(DE)**

(72) Erfinder: **Elbe, Hans-Ludwig, Dr.**
**Dasnöckel 59**
**D-5600 Wuppertal 11(DE)**

(54) **Ein Verfahren zur Herstellung von optisch aktiven Phenoxythiopropionsäure-Derivaten und neue Zwischenprodukte zu ihrer Herstellung.**

(57) Die Erfindung betrifft ein neues Verfahren zur Herstellung von bekannten herbiziden optisch aktiven Phenoxythiopropionsäure-Derivaten der Formel (I)

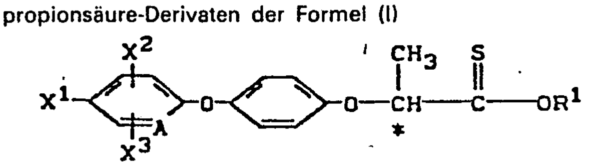

(Substituenten-Definition siehe Beschreibung) durch Umsetzung von geeigneten optisch aktiven Imidiumsäureestern mit Schwefelwasserstoff.

Die Erfindung betrifft weiterhin neue Zwischenprodukte, welche für die Durchführung des Verfahrens verwendet werden, sowie Verfahren zur Herstellung solcher Zwischenprodukte.

EP 0 204 231 A2

BAYER AKTIENGESELLSCHAFT 5090 Leverkusen, Bayerwerk
Konzernverwaltung RP 86107042.3
Patentabteilung KM/li-c
(IVa/Ia)ZP

# Ein Verfahren zur Herstellung von optisch aktiven Phenoxythiopropionsäure-Derivaten und neue Zwischenprodukte zu ihrer Herstellung

Die Erfindung betrifft ein neues Verfahren zur Herstellung von bekannten herbiziden optisch aktiven Phenoxythiopropionsäure-Derivaten, neue Zwischenprodukte, welche für die Durchführung des Verfahrens verwendet werden können, sowie Verfahren zur Herstellung solcher Zwischenprodukte.

Es ist bereits bekannt, daß man Phenoxyalkanthiocarbonsäure-Derivate erhält, wenn man Phenoxyalkancarbonsäure-Derivate mit Schwefelungsmitteln, wie z. B. 2,4-Bis(4-methoxy-phenyl)-1,3,2,4-dithiaphosphetan-2,4-disulfid umsetzt (vgl. EP-A 115 823).

Der Nachteil bei diesem Verfahren besteht darin, daß bei der Schwefelung von optisch aktiven Phenoxyalkancarbonsäure-Derivaten häufig Racemisierung auftritt.

Es wurde nun gefunden, daß man optisch aktive Phenoxythiopropionsäure-Derivate der Formel (I)

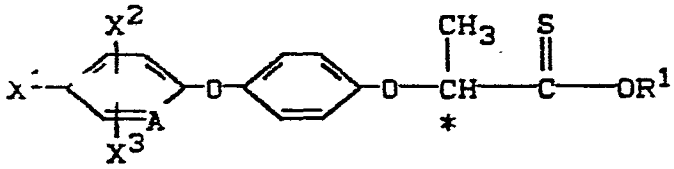 (I)

in welcher

A für Stickstoff oder eine CH-Gruppierung steht,

$X^1$ für Wasserstoff, Halogenalkyl oder Halogen steht,

$X^2$ und $X^3$ unabhängig voneinander jeweils für Wasserstoff, Halogenalkyl oder Halogen steht und

$R^1$ für Methyl oder Ethyl steht,

erhält, wenn man Salze von optisch aktiven Imidiumsäureestern der allgemeinen Formel (II)

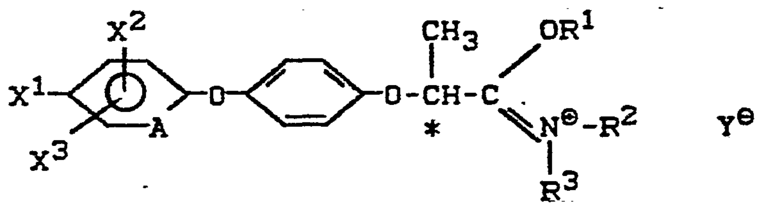 (II)

in welcher

A, $R^1$, $X^1$, $X^2$ und $X^3$ die oben angegebenen Bedeutungen haben,

$R^2$ und $R^3$ gleich oder verschieden sind und für Alkyl stehen

oder

$R^2$ und $R^3$ gemeinsam mit dem Stickstoffatom für einen heterocyclischen 5- oder 6-Ring stehen und

$Y^{\ominus}$ für ein Tetrafluoroborat-Ion, Methylsulfat-Ion oder Ethylsulfat-Ion steht,

mit Schwefelwasserstoff, in Gegenwart von Säureakzeptoren und gegebenenfalls in Gegenwart von inerten Verdünnungsmitteln bei Temperaturen zwischen -30 und +100°C umsetzt.

In den obigen Formeln und auch im folgenden Teil der Beschreibung ist das asymmetrisch substituierte Kohlenstoffatom jeweils durch ein (*) gekennzeichnet. Die Angabe "R" oder "S" bezeichnet die absolute Konfiguration am asymmetrisch substituierten Kohlenstoffatom.

Es ist als äußerst überraschend zu bezeichnen, daß sich die optisch aktiven Verbindungen der Formel (I) nach dem erfindungsgemäßen Verfahren in guter Ausbeute und Reinheit herstellen lassen, obwohl nach dem Stand der Technik zu erwarten war, daß das CH-acide Asymmetriezentrum in Gegenwart von basischen Verbindungen racemisiert. Außerdem war zu erwarten, daß die gleichzeitig bei dem erfindungsgemäßen Verfahren gebildeten sekundären Amine mit den Verbindungen der Formel (I) zu den entsprechenden Phenoxythiopropionsäureamiden weiterreagieren würden (vgl. z. B. Chem. Ber. 96, 1478 (1963)).

Das erfindungsgemäße Verfahren weist eine Reihe von Vorteilen auf. So sind die Reaktionskomponenten auch in größeren Mengen zugänglich und auch im technischen Maßstab problemlos zu handhaben. Ferner ist der zur Durchführung

des Verfahrens erforderliche apparative Aufwand gering, und die Aufarbeitung des nach beendeter Umsetzung anfallenden Reaktionsgemisches bereitet keine Schwierigkeiten. Insbesondere jedoch lassen sich die Stoffe der Formel (I) nach dem erfindungsgemäßen Verfahren nicht nur in guter Ausbeute, sondern auch frei von störenden racemischen Nebenprodukten herstellen.

Weiterhin ist vorteilhaft, daß bei dem erfindungsgemäßen Verfahren die absolute Konfiguration am asymmetrisch substituierten Kohlenstoffatom erhalten bleibt.

Mit Hilfe des erfindungsgemäßen Verfahrens lassen sich vorzugsweise optisch aktive Phenoxythiopropionsäure-Derivate der Formel (I) herstellen, in welcher

A für Stickstoff oder eine -CH-Gruppierung,

$X^1$ für Wasserstoff, Halogenalkyl mit 1 bis 4 Kohlenstoffatomen und 1 bis 5 Halogenatomen, für Fluor, Chlor, Brom oder Jod steht,

$X^2$ für Wasserstoff, Halogenalkyl mit 1 bis 4 Kohlenstoffatomen und 1 bis 5 Halogenatomen, für Fluor, Chlor, Brom oder Jod steht,

$X^3$ für Wasserstoff und Halogenalkyl mit 1 bis 4 Kohlenstoffatomen und 1 bis 5 Halogenatomen, Fluor, Chlor, Brom oder Jod steht,

$R^1$ für Methyl oder Ethyl steht.

Nach dem erfindungsgemäßen Verfahren läßt sich besonders bevorzugt die Gruppe von optisch aktiven Phenoxythiopropionsäure-Derivaten der Formel (I) herstellen, in welcher

A für eine CH-Gruppe steht,

$X^1$ für Trifluormethyl steht,

$X^2$ für Wasserstoff oder Chlor steht,

$X^3$ für Wasserstoff oder Chlor steht und

$R^1$ für Methyl oder Ethyl steht.

Eine weitere besonders bevorzugte Gruppe von Verbindungen, die nach dem erfindungsgemäßen Verfahren hergestellt werden können, sind diejenigen optisch aktiven Phenoxythiopropionsäure-Derivate der Formel (I), in denen

A für Stickstoff steht,

$X^1$ für Chlor oder Trifluormethyl steht,

$X^2$ für Wasserstoff oder Chlor steht,

$X^3$ für Wasserstoff steht und

$R^1$ für Methyl oder Ethyl steht.

Ganz besonders bevorzugt werden mit Hilfe des erfindungsgemäßen Verfahrens die R-Enantiomeren der Verbindungen der Formel (I) hergestellt.

Verwendet man das Salz aus Trimethyloxonium-tetrafluoroborat und dem R-Enantiomeren des 2-[4-(2-Trifluormethyl-5-chlor-phenoxy)-phenoxy]-propionsäure-dimethylamid und Schwefelwasserstoff als Ausgangsstoffe, so läßt sich der Reaktionsablauf des erfindungsgemäßen Verfahrens durch das folgende Formelschema darstellen:

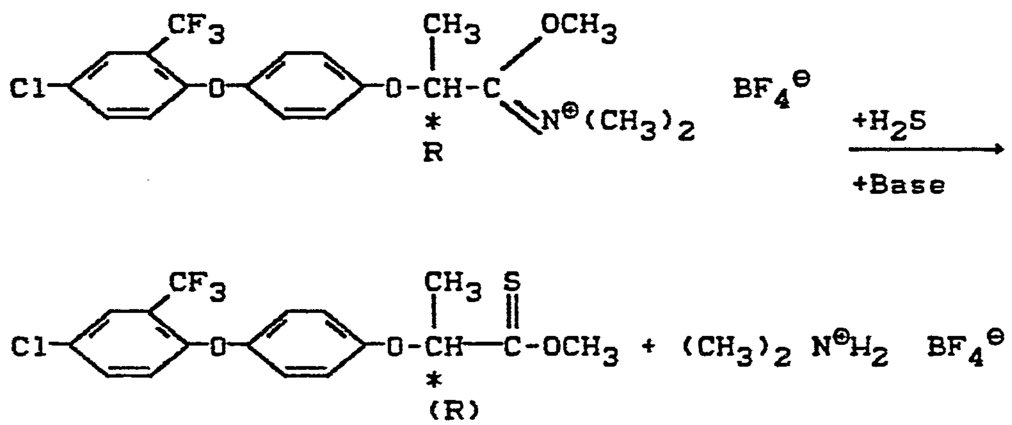


Die zur Durchführung des erfindungsgemäßen Verfahrens als Ausgangsstoffe benötigten optisch aktiven Imidiumsäureester-Salze sind durch die Formel (II) allgemein definiert. In dieser Formel (II) haben A, $R^1$, $X^1$, $X^2$ und $X^3$ bevorzugt diejenigen Bedeutungen, welche bereits bei der Definition der Formel (I) als bevorzugt für diese Substituenten genannt wurden. In dieser Formel (II) stehen weiterhin $R^2$ und $R^3$ vorzugsweise für Alkyl mit 1 bis 6 Kohlenstoffatomen oder $R^2$ und $R^3$ stehen gemeinsam mit dem Stickstoffatom, an das sie gebunden sind, vorzugsweise für einen Piperidyl- oder PyrrolidinylRest und $Y^{\ominus}$ steht für ein Tetrafluoroborat-Ion, Methylsulfat-Ion oder Ethylsulfat-Ion.

In der Formel (II) stehen A besonders bevorzugt für Stickstoff oder eine CH-Gruppe, $R^1$ für Methyl oder Ethyl und $R^2$ und $R^3$ jeweils für Alkyl mit 1 bis 4 Kohlenstoffatomen oder gemeinsam mit dem Stickstoffatom, an das sie gebunden sind, für einen Piperidyl- oder Pyrrolidinyl-Rest und $Y^{\ominus}$ für ein Tetrafluoroborat-, Methyl- oder Ethylsulfat-Ion.

In der Formel (II) stehen $X^1$, $X^2$ und $X^3$ besonders bevorzugt für Wasserstoff, Halogenalkyl mit 1 bis 2 Kohlenstoffatomen und 1 bis 3 Halogenatomen, Fluor, Chlor oder Brom. Ganz besonders bevorzugt stehen $X^1$, $X^2$ und $X^3$ für Wasserstoff, Trifluormethyl oder Chlor.

Die Salze der optisch aktiven Imidiumsäureester der Formel (II) sind erfindungsgemäße Verbindungen. Sie lassen sich auf einfache Weise herstellen, indem man optisch aktive Phenoxypropionsäureamide der Formel (III)

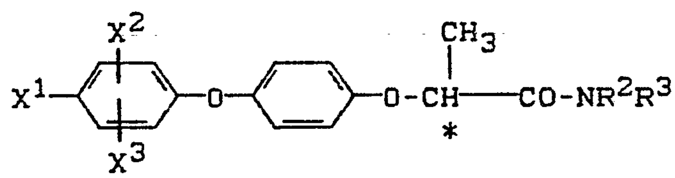

(III)

in welcher

A, $X^1$, $X^2$, $X^3$, $R^2$ und $R^3$ die oben angegebenen Bedeutungen haben,

(a) mit Oxoniumsalzen der Formel (IV)

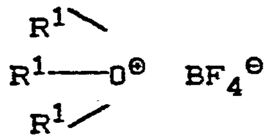 (IV)

in welcher

$R^1$ die oben angegebene Bedeutung hat,

oder

(b) mit Dialkylsulfaten der Formel (V)

$$R^1O\text{-}SO_2\text{-}OR^1 \qquad (V)$$

in welcher

$R^1$ die oben angegebene Bedeutung hat,

in Gegenwart von inerten Verdünnungsmitteln umsetzt.

Die als Ausgangsstoffe für die Herstellung der Verbindungen der Formel (II) benötigten optisch aktiven Phenoxypropionsäureamide sind durch die Formel (III) eindeutig definiert. In dieser Formel haben A, $X^1$, $X^2$, $X^3$, $R^2$ und $R^3$ vorzugsweise diejenigen Bedeutungen, die bereits im Zusammenhang mit der Beschreibung der Verbindungen der Formeln (I) und (II) vorzugsweise für diese Substituenten genannt wurden.

Die Verbindungen der Formel (III) sind bekannt oder lassen sich nach bekannten Methoden auf einfache Weise aus den entsprechenden optisch aktiven Phenoxypropionsäurehalogeniden durch Umsetzung mit sekundären Aminen herstellen (vgl. DE-OS 32 19 789, DE-OS 32 21 214, DE-OS 32 19 821, BE-PS 862 325, BE-PS 868 875, EP-OS 483, EP-OS 17 767, EP-OS 1 473, US-PS 4 301 295 und DE-OS 29 46 652 und Herstellungsbeispiel).

Als Beispiele für die R- und S-enantiomeren Verbindungen der Formel (III) seien genannt:

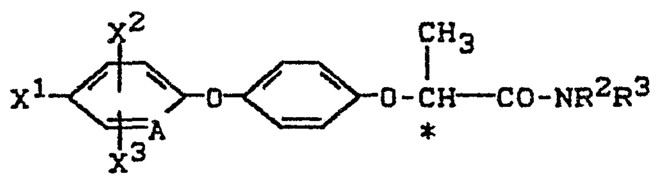

(III)

Tabelle 1

| 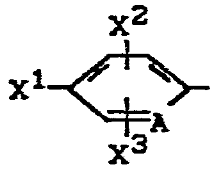 | $-NR^2R^3$ | 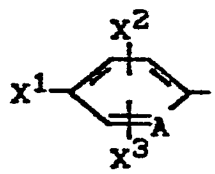 | $-NR^2R^3$ |
| --- | --- | --- | --- |
| 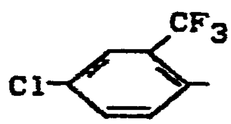 | $-N(CH_3)_2$ | 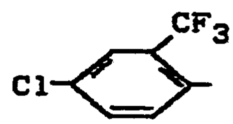 | $-N(C_2H_5)_2$ |
| 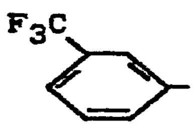 | $-N(CH_3)_2$ | 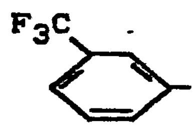 | $-N(C_2H_5)_2$ |
| 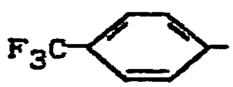 | $-N(CH_3)_2$ | 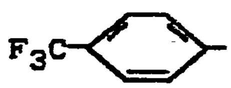 | $-N(C_2H_5)_2$ |
| 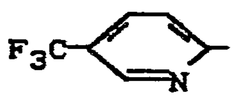 | $-N(CH_3)_2$ | 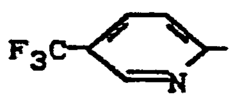 | $-N(C_2H_5)_2$ |
| 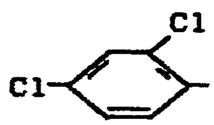 | $-N(CH_3)_2$ | 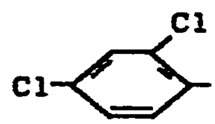 | $-N(C_2H_5)_2$ |
| 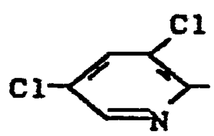 | $-N(CH_3)_2$ | 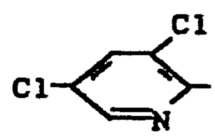 | $-N(C_2H_5)_2$ |
| 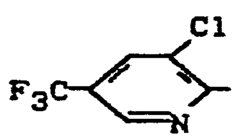 | $-N(CH_3)_2$ | 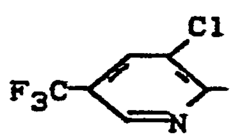 | |

Tabelle 1 - Fortsetzung

| 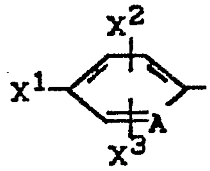 | $-NR^2R^3$ | 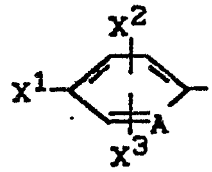 | $-NR^2R^3$ |
|---|---|---|---|
| 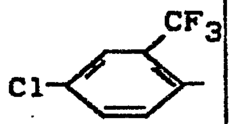 | $-N(C_3H_7-n)_2$ | 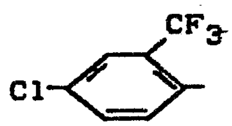 | 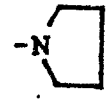 |
| 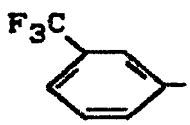 | $-N(C_3H_7-n)_2$ | 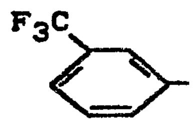 | 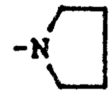 |
| 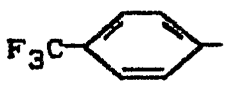 | $-N(C_3H_7-n)_2$ | 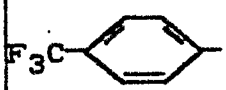 | 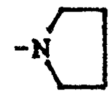 |
| 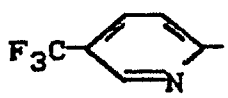 | $-N(C_3H_7-n)_2$ | 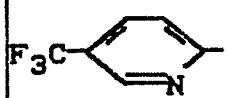 | 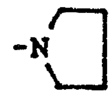 |
| 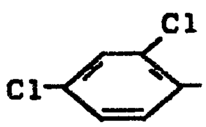 | $-N(C_3H_7-n)_2$ | 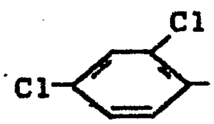 | 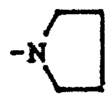 |
| 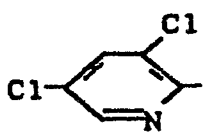 | $-N(C_3H_7-n)_2$ | 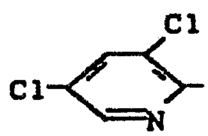 | 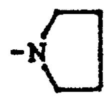 |
| 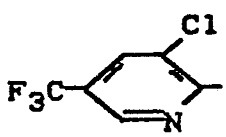 | $-N(C_3H_7-n)_2$ | 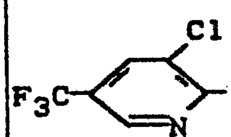 | 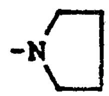 |

Tabelle 1 - Fortsetzung

| 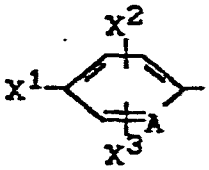 | $-NR^2R^3$ |
|---|---|
| 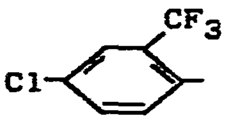 | 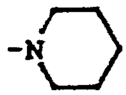 |
| 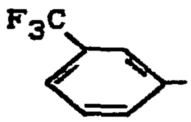 | 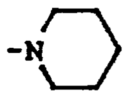 |
| 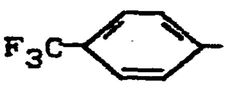 | 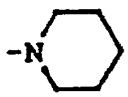 |
| 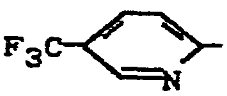 | 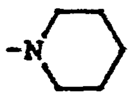 |
| 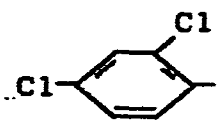 | 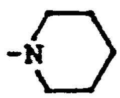 |
| 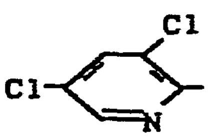 | 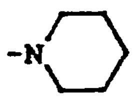 |
| 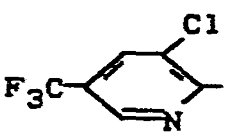 | 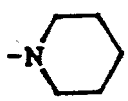 |

Die weiterhin als Ausgangsstoffe für die Herstellung der Verbindungen der Formel (II) benötigten Oxoniumsalze bzw. Dialkylsulfate sind durch die Formeln (IV) bzw. (V) eindeutig definiert. In diesen Formeln steht $R^1$ für Methyl oder Ethyl.

Die Verbindungen der Formeln (IV) bzw. (V) sind bekannte Verbindungen der organischen Chemie.

Für die Verbindungen der Formel (IV) seien genannt: Trimethyl- und Triethyloxoniumtetrafluoroborat.

Für die Verbindungen der Formel (V) seien genannt: Dimethyl- und Diethylsulfat.

Das Verfahren zur Herstellung von Verbindungen der Formel (II) wird bevorzugt in Gegenwart von inerten Verdünnungsmitteln durchgeführt.

Hierzu gehören insbesondere aliphatische und aromatische, gegebenenfalls halogenierte Kohlenwasserstoffe, wie Pentan, Hexan, Heptan, Cyclohexan, Petrolether, Benzin, Ligroin, Benzol, Toluol, Xylol, Methylenchlorid, 1,2-Dichlorethan, Chloroform, Tetrachlorkohlenstoff, Chlorbenzol und o-Dichlorbenzol.

Das Verfahren zur Herstellung der Verbindungen der Formel (II) wird im allgemeinen bei Temperaturen zwischen -20°C und +60°C durchgeführt. Bevorzugt wird der Bereich zwischen -10°C und +40°C.

Die Umsetzungen werden im allgemeinen bei Normaldruck durchgeführt.

Bei der Durchführung des Verfahrens zur Herstellung der Verbindungen der Formel (II) werden die Ausgangsstoffe im allgemeinen in etwa äquimolaren Mengen eingesetzt. Es ist jedoch auch möglich, eine der beiden Komponenten in einem größeren Überschuß einzusetzen. Die Reaktion wird im allgemeinen in einem geeigneten Verdünnungsmittel durchgeführt, und das Reaktionsgemisch wird mehrere Stunden bei der erforderlichen Temperatur gerührt. Die Aufarbeitung erfolgt bei diesem Verfahren nach üblichen Methoden.

Das erfindungsgemäße Verfahren zur Herstellung der Verbindungen der Formel (I) wird vorzugsweise unter Verwendung von Verdünnungsmitteln durchgeführt. Als Verdünnungsmittel kommen dabei praktisch alle inerten organischen Lösungsmittel in Frage. Hierzu gehören vorzugsweise Ether wie Diethyl-, Diisopropyl- und Dibutylether, Glykoldimethylether und Diglykoldimethylether, Tetrahydrofuran und Dioxan.

Das erfindungsgemäße Verfahren wird in Gegenwart von Säureakzeptoren durchgeführt. Als Säureakzeptoren können aliphatische, aromatische oder heterocyclische Amine wie beispielsweise Triethylamin, Trimethylamin, Tripropylamin, Dimethylanilin, Dimethylbenzylamin, Dimethylcyclohexylamin und Pyridin verwendet werden.

Das erfindungsgemäße Verfahren wird im allgemeinen bei Temperaturen zwischen -30° C und +100° C durchgeführt. Bevorzugt wird der Bereich zwischen -20° C und + 50° C, besonders bevorzugt wird der Bereich zwischen +20° C und +35° C. Die Umsetzungen werden im allgemeinen bei Normaldruck durchgeführt.

0204231

Bei der Durchführung des erfindungsgemäßen Verfahrens setzt man auf 1 Mol Imidiumsäureester-Salz der Formel (II) 1 bis 5 Mol, vorzugsweise 1,5 bis 3,0 Mol Schwefelwasserstoff und 1 bis 2 Mol, vorzugsweise 1 bis 1,5 Mol Säureakzeptor ein.

Die Reaktion wird bevorzugt in einem geeigneten Verdünnungsmittel durchgeführt und das Reaktionsgemisch wird mehrere Stunden bei der erforderlichen Temperatur gerührt. Die Aufarbeitung erfolgt nach üblichen Methoden.

Viele der erfindungsgemäß erhältlichen Verbindungen und ihre herbizide Verwendung sind bekannt (vgl. DE-OS 3 304 204).

## Herstellungsbeispiele

### Beispiel 1

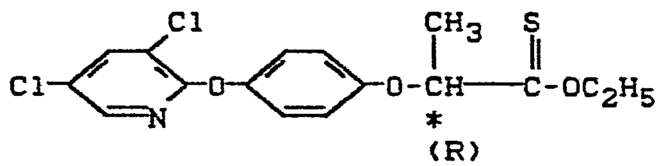

150 ml Tetrahydrofuran im Gemisch mit 5 ml Pyridin wird bei 0°C mit Schwefelwasserstoff gesättigt. Zu dieser Lösung gibt man 26,6 g (0,056 Mol) des Salzes aus Triethyloxonium-tetrafluoroborat und dem R-Enantiomeren des 2-[4-(3,5- Dichlor-pyrid-2-yl-oxy)-phenoxy]-propionsäure-dimethylamids und leitet bei 0°C weiter Schwefelwasserstoff ein, bis die Lösung erneut mit Schwefelwasserstoff gesättigt ist (Dauer ca. 4 Stunden).Nach beendeter Reaktion versetzt man das Reaktionsgemisch mit Ether, wäscht die Etherphase mit verdünnter Salzsäure und anschließend mit Wasser. Dann wird die organische Phase über Natriumsulfat getrocknet und eingeengt.

Das Rohprodukt (21,9 g) wird durch Säulenfiltration gereinigt (Kieselgel/Essigester-Hexan im Verhältnis 3:1).

Man erhält 11 g (52 % der Theorie) an R-Enantiomeren des 2-[4-(3,5-Dichlor-pyrid-2-yloxy)-phenoxy]-thiopropionsäureethylesters.

R/S-Verhältnis = 96:4

Drehwert: $[\alpha]_D^{2°} = + 44{,}9°$

(1-molare Lösung in Chloroform; Küvettenlänge 10 cm).

Beispiel (II-1)

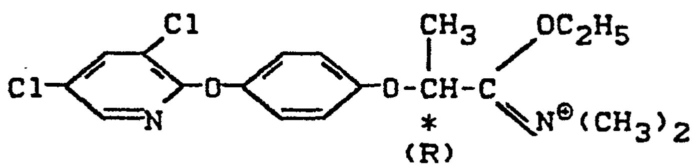

$BF_4^{\ominus}$ (II-1)

45 g (0,126 Mol) an R-Enantiomeren des 2-[4-(3,5-Dichlor-pyrid-2-yl-oxy)-phenoxy]-propionsäure-dimethylamids wird in 80 ml Methylenchlorid vorgelegt. Bei 20°C werden 18,6 g (0,126 Mol) Triethyloxonium-tetrafluoroborat zugegeben und 3 Stunden bei 20°C gerührt. Anschließend wird 2 Stunden unter Rückfluß weitergerührt. Zur Aufarbeitung wird das Lösungsmittel unter vermindertem Druck entfernt.

Man erhält 59,0 g rohes Salz aus Triethyloxonium-tetrafluoroborat und dem R-Enantiomeren des 2-[4-(3,5-Dichlor-pyrid-2-yl-oxy)-phenoxy]-propionsäure-dimethylamids als hochviskoses Öl. Das Rohprodukt kann ohne weitere Reinigung zur Herstellung der Verbindungen der Formel (I) eingesetzt werden.

Beispiel (III-1)

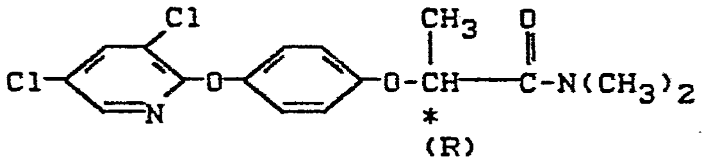

346,5 g 2-[4-(3,5-Dichlor-pyrid-2-yl-oxy)-phenoxy]-propionsäurechlorid (R/S-Verhältnis = 87:13) werden in 1500 ml Toluol vorgelegt und bei einer Temperatur von 10°C bis 20°C innerhalb von 30 Minuten mit 337,5 g 40 %iger Dimethylaminlösung versetzt und 30 Minuten bei 20°C nachgerührt. Das Gemisch wird anschließend mit 1000 ml Wasser extrahiert. Die organische Phase wird mit Wasser nachgewaschen, getrocknet und eingeengt.

Man erhält 308 g (87 % der Theorie) an 2-[4-(3,5-Dichlor-pyrid-2-yl-oxy)-phenoxy]-propionsäure-dimethylamid als R- und S-Enantiomeren-Gemisch.

Zur Abtrennung des S-Enantiomeren wird das Enantiomerengemisch mit 500 ml Ether verrührt. Das schwerlösliche Racemat fällt aus.

Man erhält auf diese Weise 60 g Racemat vom Schmelzpunkt 140°C und 248 g (70% der Theorie) an R-Enantiomeren des 2-[4-(3,5-Dichlor-pyrid-2-yl-oxy)-phenoxy]-propionsäure-dimethylamids vom Brechungsindex $n_D^{20}$: 1,5814.

Drehwert: $[\alpha]_D^{20} = + 3{,}04°$
(1-molare Lösung in Chloroform;
Küvettenlänge 10 cm)

0204231

Patentansprüche

1. Verfahren zur Herstellung von optisch aktiven Phenoxythiopropionsäure-Derivaten der Formel (I),

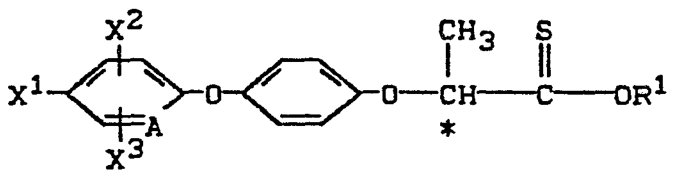

(I)

in welcher

A für Stickstoff oder eine CH-Gruppierung steht,

$X^1$ für Wasserstoff, Halogenalkyl oder Halogen steht,

$X^2$ und $X^3$ unabhängig voneinander jeweils für Wasserstoff, Halogenalkyl oder Halogen stehen und

$R^1$ für Methyl oder Ethyl steht,

dadurch gekennzeichnet, daß man Salze von optisch aktiven Imidiumsäureestern der Formel (II),

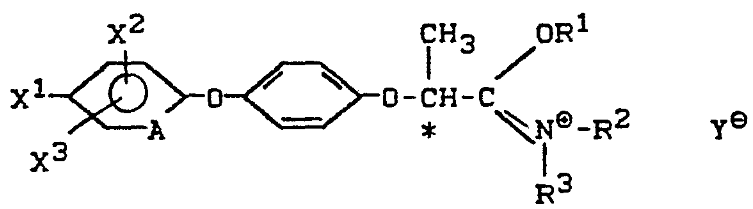

(II)

in welcher

A, $R^1$, $X^1$, $X^2$ und $X^3$ die oben angegebene Bedeutung haben,

$R^2$ und $R^3$ gleich oder verschieden sind und für Alkyl stehen

oder

$R^2$ und $R^3$ gemeinsam mit dem Stickstoffatom für einen heterocyclischen 5- oder 6-Ring stehen und

$Y^\ominus$ für ein Tetrafluoroborat-Ion, Methylsulfat-Ion oder Ethylsulfat-Ion steht,

mit Schwefelwasserstoff, in Gegenwart von Säureakzeptoren und gegebenenfalls in Gegenwart von inerten Verdünnungsmitteln bei Temperaturen zwischen -30 und +100°C umsetzt.

2. Verfahren nach Anspruch 1, dadurch gekennzeichnet, daß man die Umsetzung bei Temperaturen zwischen -20 und +50°C durchführt.

3. Verfahren nach Anspruch 1, dadurch gekennzeichnet, daß man auf 1 Mol Imidiumsäureester-Salz der Formel (II) 1 bis 5 Mol Schwefelwasserstoff einsetzt.

4. Verfahren nach Anspruch 3, dadurch gekennzeichnet, daß man auf 1 Mol Imidiumsäureester-Salz der Formel (II) 1,5 bis 3,0 Mol Schwefelwasserstoff einsetzt.

5. Verfahren nach Anspruch 1, dadurch gekennzeichnet, daß man auf 1 Mol Imidiumsäureester-Salz der Formel (II) 1 bis 2 Mol Säureakzeptor einsetzt.

6. Verfahren nach Anspruch 5, dadurch gekennzeichnet, daß man als Säureakzeptor Amine einsetzt.

7. Verfahren nach Anspruch 1, dadurch gekennzeichnet, daß man in Gegenwart eines Verdünnungsmittels arbeitet.

8. Salze der optisch aktiven Imidiumsäureester der Formel (II),

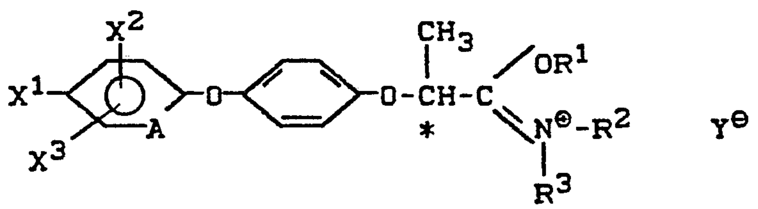

(II)

in welcher

A für Stickstoff oder eine CH-Gruppierung steht,

$X^1$ für Wasserstoff, Halogenalkyl oder Halogen steht,

$X^2$ und $X^3$ unabhängig voneinander jeweils für Wasserstoff, Halogenalkyl oder Halogen stehen und

$R^1$ für Methyl oder Ethyl steht,

$R^2$ und $R^3$ gleich oder verschieden sind und für Alkyl stehen

oder

$R^2$ und $R^3$ gemeinsam mit dem Stickstoffatom für einen heterocyclischen 5- oder 6-Ring stehen und

$Y^{\ominus}$ für ein Tetrafluoroborat-Ion, Methylsulfat-Ion oder Ethylsulfat-Ion steht.

9. Salze der optisch aktiven Imidiumsäureester der Formel (II) gemäß Anspruch 8, in welcher

A für Stickstoff oder eine CH-Gruppierung steht,

$X^1$ für Wasserstoff, Halogenalkyl mit 1 bis 4 Kohlenstoffatomen und 1 bis 5 Halogenatomen, Fluor, Chlor, Brom oder Jod steht,

$X^2$ für Wasserstoff, Halogenalkyl mit 1 bis 4 Kohlenstoffatomen und 1 bis 5 Halogenatomen, Fluor, Chlor, Brom oder Jod steht,

$X^3$ für Wasserstoff, Halogenalkyl mit 1 bis 4 Kohlenstoffatomen und 1 bis 5 Halogenatomen, Fluor, Chlor, Brom oder Jod steht,

$R^1$ für Methyl oder Ethyl steht,

$R^2$ und $R^3$ gleich oder verschieden sind und für Alkyl mit 1 bis 6 Kohlenstoffatomen stehen

oder

$R^2$ und $R^3$ gemeinsam mit dem Stickstoffatom für einen Piperidyl- oder Pyrrolidinyl-Rest stehen und

$Y^\ominus$ für ein Tetrafluoroborat-Ion, Methylsulfat-Ion oder Ethylsulfat-Ion steht.

10. Verfahren zur Herstellung von Salzen der optisch aktiven Imidiumsäureester der Formel (II),

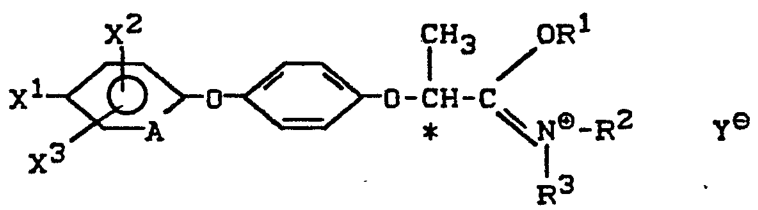

(II)

in welcher

A für Stickstoff oder eine CH-Gruppierung steht,

$X^1$ für Wasserstoff, Halogenalkyl oder Halogen steht,

$X^2$ für Wasserstoff, Halogenalkyl oder Halogen steht,

$X^3$ für Wasserstoff, Halogenalkyl oder Halogen steht,

$R^1$ für Methyl oder Ethyl steht,

$R^2$ und $R^3$ gleich oder verschieden sind und für Alkyl stehen

oder

$R^2$ und $R^3$ gemeinsam mit dem Stickstoffatom für einen heterocyclischen 5- oder 6-Ring stehen und

$Y^\ominus$ für ein Tetrafluoroborat-Ion, Methylsulfat-Ion oder Ethylsulfat-Ion steht,

dadurch gekennzeichnet, daß man optisch aktive Phenoxypropionsäureamide der Formel (III),

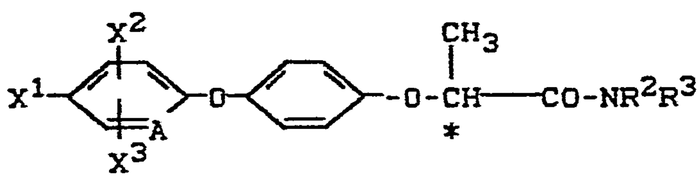

(III)

in welcher

A, $X^1$, $X^2$, $X^3$, $R^2$ und $R^3$ die oben angegebenen Bedeutungen haben,

a) mit Oxoniumsalzen der Formel (IV)

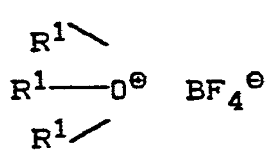

(IV)

0204231

in welcher

$R^1$ die oben angegebene Bedeutung hat,

oder

b) mit Dialkylsulfaten der Formel (V)

$$R^1O-SO_2-OR^1 \qquad (V)$$

in welcher

$R^1$ die oben angegebene Bedeutung hat,

in Gegenwart von inerten Verdünnungsmittel umsetzt.